# DEMANDE DE BREVET EUROPEEN

(11) **EP 3 150 225 A1**
(43) Date de publication de la demande: **05.04.2017**
(21) Numéro de dépôt: 16197817.6
(22) Date de dépôt: 18.07.2011
(51) Int. Cl.: A61K 39/395, C07K 16/06, A61K 47/00

(54) **COMPOSITION D'IMMUNOGLOBULINES HUMAINES CONCENTREES**

(30) Priorité: 19.07.2010 FR 1055825
(62) Demande divisionnaire de: 11754440.3
(71) Demandeur: Laboratoire Français du Fractionnement et des Biotechnologies, 91940 Les Ulis (FR)
(72) Inventeur: HUILLE, Sylvain, 92160 ANTONY (FR); COHEN-TANNOUDJI, Laetitia, 75014 PARIS (FR); ARVIS, Florence, 92100 BOULOGNE-BILLANCOURT (FR); PAILLARD, Alexandra, 91942 LES ULIS (FR)
(74) Mandataire: Cabinet Becker et Associés

(57) **Abrégé**

L'invention concerne une composition d'immunoglobulines G humaines caractérisée en ce que la concentration en immunoglobulines G humaines est d'au moins 230g/L, utile notamment pour une administration par la voie sous-cutanée.

## Description

L'invention a trait à la formulation d'immunoglobulines G humaines, utiles en thérapie.

De nombreuses pathologies sont actuellement traitées par des compositions d'immunoglobulines G (IgG). On peut citer par exemple les déficits immunitaires primitifs avec défaut de production d'anticorps, la maladie de Kawasaki, le purpura thrombopénique immunologique de l'enfant et de l'adulte, les déficits immunitaires secondaires avec défaut de production d'anticorps, en particulier la leucémie lymphoïde chronique ou myélome associés à des infections à répétition, l'infection de l'enfant par le VIH associé à des infections bactériennes, les neuropathies motrices multifocales, le syndrome de Guillain-Barré, les infections aiguës sévères ou chroniques à Parvovirus B19, l'immunodéficience acquise ou constitutionnelle, la dermatomyosite cortico-résistante, la myasthénie aiguë, la polyradiculonévrite chronique idiopathique, le purpura thrombopénique immunologique, par exemple associé à l'infection par le VIH, le syndrome de l'homme raide (Stiffman syndrome), la neutropénie auto-immune, l'érythroblastopénie auto-immune résistante, le syndrome d'anti-coagulation acquise par auto-anticorps, la polyarthrite rhumatoïde, les uvéites, etc.

Les pathologies traitées par les Immunoglobulines engagent des posologies particulièrement élevées qui représentent des doses de l'ordre de 0.4 à 2 grammes par poids corporel du patient et par mois. Dans ce contexte la majorité des immunoglobulines G commercialisées aujourd'hui sont destinées à une administration par voie intraveineuse qui autorise des perfusions de plusieurs dizaines de grammes d'IgG dans des préparations de plusieurs centaines de millilitres à raison d'une administration toutes les 3 ou 4 semaines. L'administration par voie intraveineuse requiert la présence de personnel soignant ; elle est le plus souvent réalisée à l'hôpital. Chez les jeunes enfants et les personnes âgées, l'administration par voie intraveineuse peut être rendue très difficile en raison d'un mauvais abord veineux, qui peut dans certains cas, empêcher l'accès au traitement.

Il existe des IgG destinées à l'administration sous-cutanée (IgSC). Cette voie d'administration offre plus de flexibilité et d'indépendance au patient, améliorant la qualité de vie des patients. De plus, l'usage des immunoglobulines sous-cutanées (IgSC) réduit également certains effets secondaires associés aux perfusions en intraveineuse, en particulier le risque de réactions systémiques. Les grandes variations de taux circulants observées par voie intraveineuse sont évitées par voie sous-cutanée permettant une meilleure régulation du taux sérique dans la fourchette physiologique entre les perfusions.

Une des principales limitations de l'emploi des IgSC est la dose à administrer : elle requiert des volumes importants injectés en un ou plusieurs sites à une fréquence plus resserrée que les IgIV, toutes les 2 semaines ou toute les semaines ou voire même plusieurs fois par semaines. L'injection de volumes importants avec les IgSC commerciales peut provoquer des réactions d'intolérance locale tels que des oedèmes ou des érythèmes (M. Delire et al., "Expérience clinique de l'administration d'immunoglobulines par voie sous-cutanée dans le traitement des immunodéficiences primaires", Rev Med Liège 2010;65:2;103-108). La concentration de l'IgSC est une caractéristique déterminante qui conditionne le volume d'injection et le nombre de sites d'injection. Compte tenu des doses injectées et du volume maximum injectable par site, la concentration de l'IgSC définit le nombre de site d'injection et en conséquence la fréquence d'administration.

On connait une solution d'IgG concentrée à 200g/l ou 20% : IgPro20® d'Hizentra, qui peut être administrée par la voie sous cutanée.
Par ailleurs, il existe également d'autres solutions d'IgG concentrées à 160 g/l ou 16% et administrables par voie sous cutanée, comme notamment Vivaglobulin® de Baxter ou encore Gammanorm® d'Octapharma.

L'obtention d'une composition d'IgG hautement concentrées qui soit stable et bien tolérée pour l'administration sous-cutanée, nécessite de prendre en compte des contraintes techniques importantes.
En effet, il est notamment connu que lorsque l'on augmente la concentration en immunoglobuline d'une composition d'IgG, il peut se former des oligomères et polymères dans ladite composition. Les oligomères et polymères sont susceptibles d'activer le système du complément avec des risques associés de réactions anaphylactiques. Ces oligomères et polymères sont également susceptibles d'induire des phénomènes d'hypotension chez le patient traité. Ceci n'est pas souhaitable et est strictement contrôlé du point de vue règlementaire.
De plus, il est également connu que les solutions hautement concentrées de protéines soulèvent aussi des problèmes d'instabilité induits par l'agrégation. Contrairement aux réactions chimiques qui sont généralement indépendantes de la concentration, l'agrégation, impliquant des collisions bi-moléculaires dépend directement de la concentration. Toute exposition à un stress thermique, mécanique ou chimique pourra induire une agrégation. Par exemple, les interactions à l'interface eau-air, les contraintes mécaniques du procédé d'ultrafiltration (micro-cavitations) et aussi les modifications de force ionique sont connues pour induire une agrégation protéique.
Une autre contrainte technique importante est liée au procédé d'ultrafiltration tangentielle couramment utilisé dans le procédé de fabrication des IgG : à haute concentration en IgG une surconcentration locale des IgG au niveau des membranes peut perturber la couche de polarisation rendant ainsi moins efficace l'ultrafiltration tangentielle ou carrément induire le colmatage de la membrane en bloquant l'ultrafiltration tangentielle.
L'augmentation de la viscosité associée à la concentration en IgG est aussi une contrainte technique majeure. En réduisant l'écoulement des IgG concentrées, la viscosité peut en effet générer des problèmes tant au niveau du procédé pour les étapes de formulation et de répartition aseptique que dans l'application finale vis-à-vis de la seringabilité de la composition IgG.

Dans ce contexte, il existe un besoin grandissant de produire des compositions d'IgG à des concentrations hautement élevées pour en réduire le volume d'injection, qui sont facilement administrables et bien tolérées pour l'administration par voie sous cutanée, à partir par exemple de plasmas humains, permettant d'améliorer le confort des patients et de réduire les effets secondaires.

### Résumé de l'invention :

Afin de palier aux problèmes générés par l'obtention d'IgG hautement concentrées, la Demanderesse a mis au point un nouveau procédé permettant d'obtenir des compositions d'IgG hautement concentrées, à au moins 230g/L, plus généralement entre 230 et 350g/L, et faciles à administrer par voie sous cutanée. De manière préférée, le nouveau procédé permet d'obtenir des compositions d'IgG concentrées à au moins 250g/L, de préférence d'environ 250g/L à environ 300g/L.

Plus particulièrement le procédé de préparation d'une composition pharmaceutique comprenant des Immunoglobulines G humaines (IgG), comprend les étapes suivantes:
a) Fournir une préparation d'IgG;
b) Ajouter à la préparation, au moins un tensioactif et/ou au moins un acide aminé;
c) Procéder à une concentration des IgG par ultrafiltration,
d) Puis ajouter un tensioactif, qui peut être identique ou différent du tensioactif de l'étape b).

On peut ajouter en outre à l'étape b) un ou plusieurs composés choisis parmi les sucres, les dérivés de sucres, et les sels.

De manière préférée, le nouveau procédé de préparation d'une composition pharmaceutique comprenant des Immunoglobulines G humaines (IgG), comprenant les étapes suivantes:
a) Fournir une préparation d'IgG purifiée à partir d'une fraction plasmatique de sang humain ;
b) Ajouter à la préparation, au moins un tensioactif et/ou au moins un acide aminé, et éventuellement un ou plusieurs composés choisis parmi les sucres, les dérivés de sucres, et les sels, ledit tensioactif étant de préférence ajouté à une concentration inférieure à la concentration micellaire critique dudit tensio-actif;
c) Procéder à une concentration des IgG par ultrafiltration,
d) Puis ajouter un tensioactif, qui peut être identique ou différent du tensioactif qui sera ajouté à l'étape b), afin d'obtenir la composition pharmaceutique liquide désirée.

Les excipients de l'étape b) du procédé selon l'invention (les acides aminés, les sucres, les dérivés de sucres, les sels et/ou les tensioactifs à une concentration inférieure à la concentration micellaire critique dudit tensio-actifs) sont ajoutés avant l'étape de concentration par ultrafiltration.

Avantageusement, on ajoute à l'étape b) du procédé de préparation d'une composition pharmaceutique liquide au moins un acide aminé qui peut être un acide aminé hydrophile ou portant une chaîne latérale chargée positivement.
En particulier, on ajoute à l'étape b) du procédé de préparation d'une composition pharmaceutique liquide au moins un acide aminé, ledit acide aminé est un acide aminé hydrophile ou portant une chaîne latérale chargée positivement, associé à au moins un acide aminé hydrophobe.

Avantageusement, on ajoute à l'étape b) du procédé de préparation d'une composition pharmaceutique liquide au moins un sucre ou un dérivé de sucre choisi parmi : le saccharose, les di- et tri-saccharides, les polysaccharides, tels que le dextrose, le lactose, le maltose, le trehalose, les cyclodextrines, les maltodextrines et les dextrans, les sucres réducteurs ou les polyols.
Avantageusement, on ajoute à l'étape b) un sel choisi parmi un sel minéral et un sel organique.
Avantageusement, le tensioactif ajouté à l'étape b) et/ou d) est de préférence un détergent non ionique.

Un autre objet de l'invention concerne une composition pharmaceutique comprenant des immunoglobulines G humaines (IgG) obtenue par ce procédé, caractérisée en ce que la concentration en IgG est d'au moins 230g/L de la composition. De manière préférée, la concentration en IgG est d'au moins 250g/L de la composition.

La composition selon l'invention est avantageusement sous forme liquide.

Selon un mode de réalisation, la composition selon l'invention comprend un acide aminé et un tensioactif.
Selon un autre mode de réalisation, la composition selon l'invention comprend un acide aminé, un sel et un tensioactif.
Selon un autre mode de réalisation, la composition selon l'invention comprend un sucre ou un dérivé de sucre et un tensioactif.
Selon un autre mode de réalisation, la composition selon l'invention comprend un acide aminé, un sucre ou un dérivé de sucre, et un tensioactif.
Selon un autre mode de réalisation, la composition selon l'invention comprend un acide aminé, un sucre ou un dérivé de sucre, un sel et un tensioactif.

La composition obtenue ou susceptible d'être obtenue par le procédé décrit ici, fait également partie de l'invention.

Ces compositions sont avantageusement sous une forme adaptée pour une administration sous cutanée ou intramusculaire, de préférence une administration sous-cutanée.

### Description détaillée:

### Définitions

On entend par " Immunoglobulines G humaines » ou « IgG humaines » dans le cadre de l'invention, des immunoglobulines polyvalentes qui sont essentiellement des IgG, incluant éventuellement des IgM. Il peut s'agir d'immunoglobulines entières, ou de fragments tels que F(ab')2 ou F(ab) et toute fraction intermédiaire obtenue au cours du procédé de fabrication des immunoglobulines polyvalentes.

Le terme « stabilité » correspond à la stabilité physique et/ou chimique des IgG. Le terme « stabilité physique » se réfère à la réduction ou l'absence de formation d'agrégats insolubles ou solubles des formes dimériques, oligomériques ou polymériques des Ig, ainsi qu'à la réduction ou l'absence de toute dénaturation structurale de la molécule.

Le terme "stabilité chimique" se réfère à la réduction ou l'absence de toute modification chimique des IgG pendant le stockage, à l'état solide ou sous forme dissoute, dans des conditions accélérées. Par exemple, les phénomènes d'hydrolyse, déamination, et/ou oxydation sont évités ou retardés. L'oxydation des acides aminés contenant du soufre est limitée.

### Procédé de fabrication:

Plus particulièrement il est décrit ici le procédé de préparation d'une composition pharmaceutique comprenant des Immunoglobulines G humaines (IgG), comprend les étapes suivantes:
   a) Fournir une préparation d'IgG;
   b) Ajouter à la préparation, un ou plusieurs composés choisis parmi : les acides aminés, les sucres, les dérivés de sucres, les sels et les tensioactifs, lesdits tensioactifs étant ajoutés à une concentration inférieure à la concentration micellaire critique desdits tensio-actifs;
   c) Procéder à une concentration des IgG par ultrafiltration,
   d) Puis ajouter un tensioactif, qui peut être identique ou différent du tensioactif de l'étape b).

Plus particulièrement les inventeurs ont découvert qu'ajouter du tensioactif avant l'ultrafiltration, et de préférence fractionner le tensioactif entre l'étape b) et l'étape d), permet d'éviter une dégradation des immunoglobulines.

Dans le cadre de l'invention, les compositions d'IgG liquides signifient des solutions aqueuses de compositions d'IgG, directement obtenues par fractionnement du plasma humain. Le milieu aqueux est composé d'eau pour préparation injectable (eau PPI) pouvant contenir des excipients pharmaceutiquement acceptables et compatibles avec les IgG. Les compositions d'IgG peuvent au préalable subir des étapes spécifiques d'inactivation/élimination de virus, tel qu'un traitement solvant détergent, une pasteurisation et/ou une nanofiltration. La composition selon l'invention comprend des IgG qui peuvent être polyclonales ou monoclonales. Les IgG peuvent être isolées à partir du sang humain ou animal ou produites par d'autres moyens, par exemple par des techniques de biologie moléculaire, par exemple dans des systèmes cellulaires bien connus de l'homme du métier. La composition selon l'invention est particulièrement adaptée aux IgG hautement purifiées. Avantageusement, les IgG de la présente invention sont obtenues par fractionnement du plasma humain. Des méthodes de fractionnement préférées du plasma humain sont décrites par Cohn et al (J. Am. Chem. Soc., 68, 459, 1946), Kistler et al. (Vox Sang., 7, 1962, 414-424), Steinbuch et al (Rev. Franç. Et. Clin. et Biol., XIV, 1054, 1969) et dans la demande de brevet WO 94/9334, ces documents étant incorporés par référence dans leur globalité. Une méthode de préparation d'une composition d'immunoglobulines G est également décrite dans la demande de brevet WO 02/092632, incorporée par référence dans sa globalité.

Les concentrés d'IgG sont généralement soumis à une étape ultérieure de concentration par ultrafiltration tangentielle, puis à une filtration stérilisante et peuvent être conditionnés dans des flacons et conservés de préférence à des températures voisines de 4°C. Selon un autre mode de réalisation, les IgG de la présente invention peuvent être déplétées en anticorps anti-A et anticorps anti-B comme indiqué dans la demande de brevet WO2007/077365.

L'étape de concentration par ultrafiltration tangentielle selon l'invention permet d'atteindre une concentration en immunoglobulines d'au moins 230g/L, de préférence au moins 250g/l sans entrainer le colmatage de la membrane, l'intégrité des immunoglobulines étant maintenue tout en évitant l'agrégation aux interfaces et la dénaturation sous les contraintes d'écoulement ou d'effet de cisaillement.

Le choix des excipients repose sur leur capacité stabilisante et leur capacité à permettre la concentration des IgG lors de l'étape d'ultrafiltration tangentielle.

Les excipients sont ajoutés de la manière suivante :
On ajoute à une préparation d'immunoglobulines G humaines, purifiée à partir d'une fraction plasmatique de sang humain, des excipients qui sont au moins un acide aminé et/ou au moins un tensio-actif. De préférence le ou les tensioactifs sont ajoutés à une concentration inférieure à la concentration micellaire critique desdits tensio-actifs;
Après l'ultrafiltration, on ajoute un tensioactif, qui peut être identique ou différent du tensioactif qui sera ajouté à l'étape b), afin d'obtenir la composition pharmaceutique désirée.

Ce procédé permet d'optimiser la concentration des immunoglobulines G.

De préférence, l'étape d'ultrafiltration est une étape d'ultrafiltration tangentielle, par exemple sur une membrane de seuil de coupure inférieure à 150 kD.

De manière avantageuse, les excipients ajoutés à la préparation d'IgG avant ultrafiltration comprennent ou consistent en un ou plusieurs acides aminés, et/ou un ou plusieurs tensio-actifs, et éventuellement un ou plusieurs sels.

Selon un mode de réalisation, les excipients ajoutés à la préparation d'IgG avant ultrafiltration comprennent ou consistent en un ou plusieurs acides aminés et un tensioactif.

Selon un autre mode de réalisation, on ajoute en outre à la préparation d'IgG avant ultrafiltration des excipients qui comprennent ou consistent en un ou plusieurs sucres ou dérivés de sucres.

De manière avantageuse, les excipients ajoutés à la préparation d'IgG avant ultrafiltration comprennent ou consistent en un ou plusieurs acides aminés et un ou plusieurs sucres ou dérivés de sucres et un tensioactif.

Dans un autre mode de réalisation avantageuse, les excipients ajoutés à la préparation d'IgG avant ultrafiltration comprennent ou consistent en un ou plusieurs acides aminés et un ou plusieurs sucres ou dérivés de sucres et un ou plusieurs sels.
Dans un autre mode de réalisation avantageuse, les excipients ajoutés à la préparation d'IgG avant ultrafiltration comprennent ou consistent en un ou plusieurs acides aminés et un ou plusieurs sucres ou dérivés de sucres, un ou plusieurs sels et un tensioactif.

La Demanderesse a montré de façon surprenante que des IgG hautement concentrées peuvent être obtenues en les formulant avant l'étape de concentration des IgG par ultrafiltration, grâce à l'ajout d'un acide aminé et/ou d'un tensio-actif, typiquement à une concentration inférieure à la concentration micellaire critique dudit tensio-actifs, de manière à assurer, d'une part l'obtention de la préparation directement à la formulation requise en IgG, et d'autre part, la stabilité, la compatibilité et la bonne tolérance de la composition pharmaceutique, évitant en outre les phénomènes de colmatage au niveau de la membrane d'ultrafiltration.

Les acides aminés pouvant être ajoutés lors de l'étape b) du procédé selon l'invention sont sélectionnés parmi le groupe suivant : un acide aminé hydrophile ou portant une chaîne latérale chargée positivement, et éventuellement aussi au moins un acide aminé hydrophobe. Les acides aminés hydrophiles (ou polaires) ou les acides aminés portant une chaîne latérale chargée positivement incluent la Lysine, l'Arginine, l'Histidine, la Glycine, la Sérine, la Thréonine, la Tyrosine, l'Asparagine, la Glutamine.
Parmi les acides aminés hydrophiles ou portant une chaîne latérale chargée positivement, on peut utiliser préférentiellement la glycine ou l'histidine.
L'addition d'un acide aminé hydrophile ou portant une chaîne latérale chargée positivement tel que l'arginine, et le cas échéant d'un acide aminé hydrophobe voire d'un sel de métal alcalin, alcalino-terreux, ou d'un métal de transition, favorise la stabilisation des IgG humaines.
Les acides aminés hydrophobes (à chaîne latérale apolaire) incluent notamment les acides aminés suivants : Alanine, Valine, Leucine, Isoleucine, Phénylalanine, Tryptophane, Proline, etc.

Dans un mode de réalisation préféré, l'acide aminé de l'étape b) est de la glycine, de préférence à une concentration de 200 à 300mM.

Les sucres ou dérivés de sucres pouvant être ajoutés lors de l'étape b) du procédé selon l'invention sont sélectionnés parmi le groupe suivant : le saccharose les di- et tri-saccharides et les polysaccharides, tels que le dextrose, le lactose, le maltose, le trehalose, les cyclodextrines, les maltodextrines et les dextrans, les sucres réducteurs ou les polyols. Comme sucre réducteur, on peut citer notamment le glucose ou le fructose. Comme polyol, on peut citer notamment le mannitol, le sorbitol et le xylitol.

Par « sel », on entend un sel de métal alcalin, alcalino-terreux ou d'un métal de transition. Les sels pouvant être ajoutés lors de l'étape b) du procédé selon l'invention sont sélectionnés parmi le groupe suivant : les sels minéraux, les sels organiques ou un mélange de plusieurs sels. On peut notamment citer comme sels minéraux : le phosphate de sodium, le chlorure de sodium, le chlorure de calcium, ou le chlorure de zinc.
On peut notamment citer comme sels organiques : le citrate de sodium, le succinate de sodium, l'acétate de sodium ou le thiocyanate de sodium.
De manière préférée le sel utilisé est un sel organique, de préférence l'acétate de sodium. De manière avantageuse, on ajoute à l'étape b) de 0 à 100mM d'acétate de sodium.

L'étape b) du procédé selon l'invention peut comprendre l'ajout d'un ou plusieurs tensioactifs, par exemple de type détergent non ionique, ledit tensioactif est ajouté lors de cette étape à une concentration inférieure à la concentration micellaire critique. Un tensioactif approprié utilisé dans la composition selon l'invention est avantageusement choisi parmi le polysorbate 80 (ou Tween®80 qui est du polyoxyéthylènesorbitanne-monooléate), le polysorbate 20 (ou Tween®20 qui est du polyoyéthylènesorbitanne-monolaurate), le Triton® X 100 (octoxinol 10), poloxamères, polyoxyéthylène alkyl éthers, un bloc co-polymères d'éthylène/polypropylène et le Pluronic®F68 (polyéthylènepolypropylène glycol). De préférence, le Tween®80, le Tween®20 et le poloxamère 188 sont utilisés. Les détergents non ioniques peuvent également être combinés entre eux.

Le pH de la composition pharmaceutique est ajusté au cours de l'étape b) du procédé selon l'invention. De préférence, le pH est ajusté entre 4,0 et 8,0, avantageusement entre 4,2 et 5,5 ou entre 6,8 et 7,8. En particulier, le pH de la composition pharmaceutique liquide est compris entre 6,9 et 7,6, de préférence entre 7,0 et 7,6, de préférence entre 7,1 et 7,5, de préférence entre 7,2 et 7,4, de préférence 7,3.

L'étape d) du procédé selon l'invention peut comprendre un ou plusieurs tensioactifs, par exemple de type détergent non ionique. Un tensioactif approprié utilisé dans la composition selon l'invention est avantageusement choisi parmi le polysorbate 80 (ou Tween®80 qui est du polyoxyéthylènesorbitanne-monooléate), le polysorbate 20 (ou Tween®20 qui est du polyoyéthylènesorbitanne-monolaurate), le Triton@ X 100 (octoxinol 10), poloxamères, polyoxyéthylène alkyl éthers, un bloc co-polymères d'éthylène/polypropylène et le Pluronic®F68 (polyéthylènepolypropylène glycol). De préférence, le Tween®80, le Tween®20 et le poloxamère 188 sont utilisés. Les détergents non ioniques peuvent également être combinés entre eux. La concentration en détergent non-ionique suffisante pour stabiliser la composition selon l'invention est de préférence comprise entre 0 et 1000 ppm, de préférence entre 0 et 300 ppm, de préférence entre 0 et 50 ppm. L'ajout du tensioactif à l'étape d permet d'éviter le phénomène d'agrégation de la composition.

Selon un mode de réalisation préféré, le tensioactif est un polysorbate, de préférence du polysorbate 20 ou du polysorbate 80.

De manière préférée, le tensioactif est ajouté aux étapes b) et/ou d), à une concentration totale de 50 à 300ppm.
De manière avantageuse, le tensioactif est ajouté à l'étape b) à une concentration inférieure à 75ppm, de préférence inférieure à 50ppm, de préférence inférieure à 40ppm.

De manière surprenante, les inventeurs ont en outre mis en évidence qu'une température élevée n'était pas nécessaire, au contraire. Des résultats excellents ont été obtenus à une température inférieure à 30°C.

L'ultrafiltration de l'étape c) peut donc être réalisée à une température inférieure à 30°C, de préférence inférieure à 25°C. De préférence, l'ultrafiltration de l'étape c) est réalisée à une température entre 15°C et 25°C, de préférence environ 20°C.

Le procédé selon l'invention permet d'obtenir une composition pharmaceutique liquide caractérisée en ce que la concentration en IgG est d'au moins 230g/L dans la composition, de préférence au moins 250g/L, typiquement comprise entre 230g/L et 350g/L, dans ladite composition.

### Formulations :

La composition pharmaceutique liquide selon l'invention comprenant au moins 230 g/l d'immunoglobulines G, de préférence, elle comprend 250 g/l d'immunoglobulines G.
De manière avantageuse, la composition pharmaceutique liquide peut comprendre en outre un acide aminé et un tensioactif. En particulier, la composition pharmaceutique liquide peut comprendre un ou plusieurs acides aminés hydrophiles ou portant une chaîne latérale chargée positivement, et éventuellement aussi au moins un acide aminé hydrophobe et un tensioactif.
De manière avantageuse, la composition pharmaceutique liquide peut comprendre en outre un acide aminé, un sel et un tensioactif.
De manière avantageuse, la composition pharmaceutique liquide peut comprendre en outre un sucre ou un dérivé de sucre et un tensioactif.
De manière avantageuse, la composition pharmaceutique liquide peut comprendre en outre un acide aminé, un sucre ou un dérivé de sucre et un tensioactif.
De manière avantageuse, la composition pharmaceutique liquide peut comprendre en outre un acide aminé, un sucre ou un dérivé de sucre, un sel et un tensioactif.

Selon un mode de réalisation particulier, l'invention fournit une composition d'immunoglobulines G comprenant au moins un acide aminé, au moins un tensioactif, et éventuellement au moins un sel, caractérisée en ce que la concentration en immunoglobulines G est d'au moins 230 g/l. De préférence, la composition comprend un acide aminé, un sel et un tensioactif.

Dans un exemple de réalisation, la composition comprend :
- IgG 230 g/l;
- glycine;
- tampon acétate de sodium
- et un polysorbate, par exemple du polysorbate 80 ou 20, ou un poloxamère. le pH de la composition étant compris de préférence entre 4,2 et 5,5.

Selon un mode de réalisation particulier, l'invention fournit une composition d'immunoglobulines G comprenant au moins un acide aminé, au moins un sel et au moins un tensioactif caractérisée en ce que la concentration en immunoglobulines G est d'au moins 230 g/l. De préférence, la concentration en immunoglobulines G est d'au moins 250g/L environ et la composition comprend un acide aminé, un sel et un tensioactif.

Dans un exemple de réalisation, la composition comprend :
- IgG 250 g/l environ ;
- glycine;
- éventuellement du tampon acétate de sodium
- et un polysorbate, de préférence du polysorbate 80 ou 20, ou un poloxamère.
le pH de la composition étant compris de préférence entre 4,2 et 5,5.

Plus particulièrement, la composition peut comprendre
- environ IgG 250g/l ;
- environ 50 à 300 mM glycine;
- environ 25 à 150 mM de tampon acétate
- et environ 100 à 300 ppm de polysorbate 80 ou un poloxamère
le pH de la composition étant compris de préférence entre 4,2 et 5,5.

Selon un exemple particulier, la composition comprend
- environ IgG 250g/l ;
- environ 200 mM glycine;
- environ 50 mM de tampon acétate de sodium (soit 25mM d'acétate de sodium + 25mM d'acide acétique)
- et environ 200 ppm de polysorbate 80
le pH de la composition étant compris de préférence entre 4,2 et 5,5.

Dans un exemple préféré, la composition comprend :
- IgG à environ 250g/l ;
- environ 200 mM ou 150mM glycine;
- environ 50 mM de tampon acétate (soit 25mM d'acétate de sodium + 25mM d'acide acétique)
- et environ 200 ppm de polysorbate 80,
le pH de la composition étant compris de préférence entre 4,2 et 5,5.

Selon un autre exemple particulier, la composition comprend
- IgG 250g/l environ;
- 200 mM glycine environ;
- environ 50 mM de tampon acétate (soit 25mM d'acétate de sodium + 25mM d'acide acétique)
- et environ 200 ppm de poloxamère 188,
le pH de la composition étant compris de préférence entre 4,2 et 5,5.

Selon un mode de réalisation particulier, l'invention fournit une composition d'immunoglobulines G comprenant au moins un acide aminé et au moins un tensioactif caractérisée en ce que la concentration en immunoglobulines G est d'au moins 230 g/l. De préférence, la concentration en immunoglobulines G est d'au moins 250g/L environ et la composition comprend un acide aminé et un tensioactif.

Une composition pharmaceutique liquide préférée selon l'invention comprend
- Immunoglobulines G humaines concentrées entre 230 et 350g/L, de préférence à environ 250g/L,
- glycine ;
- éventuellement du tampon acétate de sodium;
- du polysorbate, de préférence du polysorbate 80, ou un poloxamer.
le pH de la composition étant de préférence compris entre 4,2 et 5,5.

Plus particulièrement, une composition préférée comprend :
- Immunoglobulines G humaines concentrées entre 230 et 350g/L, de préférence à environ 250g/L ;
- 200 à 300 mM de glycine ;
- 0 à 100 mM de tampon acétate de sodium;
- et 50 à 300 ppm de polysorbate.
le pH de la composition étant de préférence compris entre 4,2 et 5,5.

De manière particulière, une composition préférée comprend :
- Immunoglobulines G humaines concentrées à environ 250g/L ;
- 200 mM de glycine ;
- 50 mM de tampon acétate de sodium;
- et 200 ppm de polysorbate.
le pH de la composition étant de préférence compris entre 4,2 et 5,5.

Une autre composition préférée comprend :
- Immunoglobulines G humaines concentrées à environ 250g/L ;
- 250 mM de glycine ;
- et 200 ppm de polysorbate.
le pH de la composition étant de préférence compris entre 4,2 et 5,5.

Dans un autre exemple particulier, la composition comprend
- IgG 250g/l environ ;
- environ 200 à 250 mM histidine;
- et le cas échéant environ 100 à 300 ppm de polysorbate 80 ou un poloxamère ,
le pH de la solution étant compris entre 6,8 et 7,8.

Selon un autre exemple particulier, la composition comprend
- IgG 250g/l environ ;
- environ 200 à 250 mM arginine;
- et le cas échéant environ 100 à 300 ppm de polysorbate 80 ou un poloxamère,
le pH de la solution étant compris entre 6,8 et 7,8.

Selon un autre mode de réalisation particulier, l'invention fournit une composition d'immunoglobulines G comprenant au moins un acide aminé, au moins un sucre ou un dérivé de sucre et au moins un tensioactif caractérisée en ce que la concentration en immunoglobulines G est d'au moins 230 g/l. De préférence, la concentration en immunoglobulines G est d'au moins 250g/L environ et la composition comprend un acide aminé, un sucre et un tensioactif, le pH de la solution étant compris entre 4,2 et 5,5.

Il est aussi décrit ici une composition d'immunoglobulines G comprenant au moins un sucre ou un dérivé de sucre et au moins un tensioactif caractérisée en ce que la concentration en immunoglobulines G est d'au moins 230 g/l. De préférence, la concentration en immunoglobulines G est d'au moins 250g/L et la composition comprend un sucre et un tensioactif.

Dans un exemple particulier, la composition décrite ici comprend :
- IgG 250g/l environ ;
- environ 40-50 g/L Mannitol
- et le cas échéant environ 100 à 300 ppm de polysorbate 80 ou un poloxamère ,
le pH de la solution étant compris entre 4,2 et 5,5.

Selon un mode de réalisation particulier, l'invention fournit une composition d'immunoglobulines G comprenant au moins un acide aminé, au moins un sel et au moins un tensioactif caractérisée en ce que la concentration en immunoglobulines G est d'au moins 230 g/l. De préférence, la concentration en immunoglobulines G est de 270g/L et la composition comprend un acide aminé, un sel et un tensioactif.

Dans un exemple de réalisation, la composition comprend :
- IgG 270 g/l environ ;
- glycine environ;
- tampon acétate (acétate de sodium / acide acétique)
- et le cas échéant du polysorbate 80 ou un poloxamère.

De préférence, les seuls excipients de la composition d'IgG selon l'invention sont lesdits acides aminés, sel et tensioactif (de préférence de type détergent non ionique). Une telle composition d'IgG exclusivement constituée de ces excipients (outre les IgG), présente l'avantage d'offrir une bonne stabilité, une bonne compatibilité et une bonne tolérance locale des compositions d'IgG ainsi qu'une réduction des durées et des coûts de préparation à l'échelle industrielle grâce à la présence d'un nombre minimal efficace d'excipients ainsi que la présence d'une quantité minimale efficace d'excipients

### Voies d'administration :

La composition d'IgG de l'invention est utile en thérapie, et notamment sous forme injectable, non seulement par voie intraveineuse, mais de manière plus intéressante, par voie sous-cutanée ou intramusculaire.
La voie sous-cutanée pour le traitement de maladies auto-immunes chroniques présente plusieurs avantages comme l'amélioration du confort du patient et une diminution des effets secondaires.
L'administration sous-cutanée ne nécessite pas d'accès veineux ce qui constitue, dans certains cas, un avantage décisif lorsque l'absence d'abord veineux bloque l'accès au traitement, notamment pour les jeunes enfants.
L'usage des immunoglobulines par voie sous-cutanée réduit également certains effets secondaires associés aux perfusions par voie intraveineuse, en particulier le risque de réactions systémiques. Les grandes variations de taux circulants observées par voie intraveineuse sont évitées, permettant une meilleure régulation du taux sérique dans la fourchette physiologique entre les perfusions. En dépit d'une biodisponibilité naturellement plus faible par la voie sous-cutanée, les immunoglobulines administrées par voie sous-cutanée (IgSC) ont une efficacité au moins équivalente à celle des immunoglobulines administrées par voie intraveineuse (IgIV).

Enfin, la disponibilité des IgSC pour le traitement à domicile constitue un avantage important sinon décisif pour certains traitements. Elle offre plus de flexibilité et d'indépendance au patient, améliorant la qualité de vie des patients.

L'accroissement de la concentration contribue au confort du patient en réduisant la fréquence d'injection. La concentration de l'IgSC est une caractéristique déterminante qui conditionne le volume d'injection et nombre de sites d'injection et en conséquence la fréquence d'administration.

La composition d'IgG de l'invention, sous forme liquide après un stockage durant une période de 6 mois à 25°C présente un taux de polymères bien en deça des normes fixées par la Pharmacopée Européenne (3%), avantageusement inférieur à environ 1%.

La composition de l'invention peut être une composition pharmaceutique, c'est-à-dire adaptée à un usage thérapeutique.

La composition pharmaceutique de l'invention est ainsi utile comme médicament, notamment afin de traiter les déficits immunitaires primitifs avec défaut de production d'anticorps, la maladie de Kawasaki, le purpura thrombopénique immunologique de l'enfant et de l'adulte, les déficits immunitaires secondaires avec défaut de production d'anticorps, en particulier la leucémie lymphoïde chronique ou myélome associés à des infections à répétition, l'infection de l'enfant par le VIH associé à des infections bactériennes, les neuropathies motrices multifocales, le syndrome de Guillain-Barré, les infections aiguës sévères ou chroniques à Parvovirus B19, l'immunodéficience acquise ou constitutionnelle, la dermatomyosite cortico-résistante, la myasthénie aiguë, la polyradiculonévrite chronique idiopathique, le purpura thrombopénique immunologique, par exemple associé à l'infection par le VIH, le syndrome de l'homme raide (Stiffman syndrome), la neutropénie auto-immune, l'erythroblastopénie auto-immune résistante, le syndrome d'anti-coagulation acquise par auto-anticorps, la polyarthrite rhumatoïde, les uvéites.

Les exemples suivants illustrent l'invention sans en limiter la portée.

### Exemples :

### Exemple 1 : Préparation des compositions d'I₂G 25%.

### 1.1. Préparation de la composition F1

Une composition d'IgG a été obtenue selon la méthode développée par la Demanderesse dans la demande de brevet internationale WO2007/077365.
On ajoute à la composition d'IgG obtenue 150 mM de glycine et 50 mM de NaCl et on soumet la solution d'IgG préformulée à une ultrafiltration tangentielle sur cassette à pH compris entre 4,6 et 5. On obtient ainsi une composition d'IgG formulée et concentrée à 250 g/L.

### 1.2. Préparation de la composition F2

Une composition d'IgG a été obtenue selon la méthode développée par la Demanderesse dans la demande de brevet internationale WO2007/077365.
On ajoute à la composition d'IgG obtenue 200 mM de proline et on soumet la solution d'IgG préformulée à une ultrafiltration tangentielle sur cassette à pH compris entre 4,6 et 5. On obtient ainsi une composition d'IgG formulée et concentrée à 250 g/L.

### 1.3. Préparation de la composition F3

Une composition d'IgG a été obtenue selon la méthode développée par la Demanderesse dans la demande de brevet internationale WO2007/077365.

On ajoute à la composition d'IgG obtenue 200 mM de proline et on soumet la composition d'IgG préformulée à une ultrafiltration tangentielle sur cassette à pH compris entre 4,6 et 5. On ajoute ensuite 200 ppm de tween 80 et on obtient la composition d'IgG formulée et concentrée à 250 g/L.

### 1.4. Préparation de la composition F4

Une composition d'IgG a été obtenue selon la méthode développée par la Demanderesse dans la demande de brevet internationale WO2007/077365.
On ajoute à la composition d'IgG obtenue 150 mM de glycine et 50 mM de tampon acétate (25mM d'acétate de sodium et 25mM d'acide acétique) et on soumet la composition d'IgG préformulée à une ultrafiltration tangentielle sur cassette à pH compris entre 4,6 et 5. On ajoute ensuite 200 ppm de tween 80 et on obtient la composition d'IgG formulée et concentrée à 250 g/ L.

### 1.5. Préparation de la composition F5

Une composition d'IgG a été obtenue selon la méthode développée par la Demanderesse dans la demande de brevet internationale WO2007/077365.
On ajoute à la composition d'IgG obtenue 150 mM de glycine et 50 mM de tampon acétate (25mM d'acétate de sodium et 25mM d'acide acétique) et on soumet la composition d'IgG préformulée à une ultrafiltration tangentielle sur cassette à pH compris entre 4,6 et 5. On ajoute ensuite 200 ppm de poloxamer et on obtient la composition d'IgG formulée et concentrée à 250 g/L.

### 1.6. Préparation de la composition F6

Une composition d'IgG a été obtenue selon la méthode développée par la Demanderesse dans la demande de brevet internationale WO2007/077365.
On ajoute à la composition d'IgG obtenue 150 mM de glycine et 50 mM de tampon acétate (25mM d'acétate de sodium et 25mM d'acide acétique) et on soumet la composition d'IgG préformulée à une ultrafiltration tangentielle sur cassette à pH compris entre 4,6 et 5. On obtient ainsi la composition d'IgG formulée et concentrée à 250 g/ L.

### 1.7. Préparation de la composition F7

Une composition d'IgG a été obtenue selon la méthode développée par la Demanderesse dans la demande de brevet internationale WO2007/077365.
On ajoute à la composition d'IgG obtenue 150 mM de proline et 50 mM de tampon acétate (25mM d'acétate de sodium et 25mM d'acide acétique) et on soumet la composition d'IgG préformulée à une ultrafiltration tangentielle sur cassette à pH compris entre 4,6 et 5. On ajoute ensuite 200 ppm de tween 80 et on obtient ainsi la composition d'IgG formulée et concentrée à 250 g/ L.

### 1.8. Préparation de la composition F8

Une composition d'IgG a été obtenue selon la méthode développée par la Demanderesse dans la demande de brevet internationale WO2007/077365.
On ajoute à la composition d'IgG obtenue 250 mM de proline et 5ppm de tween 80 et on soumet la composition d'IgG préformulée à une ultrafiltration tangentielle sur cassette à pH compris entre 4,6 et 5. On ajoute ensuite 195 ppm de tween 80 et on obtient ainsi la composition d'IgG formulée et concentrée à 250 g /L.

### 1.9. Préparation de la composition F9

Une composition d'IgG a été obtenue selon la méthode développée par la Demanderesse dans la demande de brevet internationale WO2007/077365.
On ajoute à la composition d'IgG obtenue 250 mM d'histidine et 5ppm de tween 80 et on soumet la composition d'IgG préformulée à une ultrafiltration tangentielle sur cassette à pH compris entre 6,8 et 7,8. On ajoute ensuite 195 ppm de tween 80 et on obtient ainsi la composition d'IgG formulée et concentrée à 250 g/L.

### 1.10. Préparation de la composition F10

Une composition d'IgG a été obtenue selon la méthode développée par la Demanderesse dans la demande de brevet internationale WO2007/077365.
On ajoute à la composition d'IgG obtenue 250 mM d'arginine et 5ppm de tween 80 et on soumet la composition d'IgG préformulée à une ultrafiltration tangentielle sur cassette à pH compris entre 6.8 et 7,8. On ajoute ensuite 195 ppm de tween 80 et on obtient ainsi la composition d'IgG formulée et concentrée à 250 g/L.

### 1.11. Préparation de la composition F11

Une composition d'IgG a été obtenue selon la méthode développée par la Demanderesse dans la demande de brevet internationale WO2007/077365.
On ajoute à la composition d'IgG obtenue 200 mM de glycine et 50 mM de tampon acétate (25mM d'acétate de sodium et 25mM d'acide acétique) et on soumet la composition d'IgG préformulée à une ultrafiltration tangentielle sur cassette à pH compris entre 4,5 et 5 On ajoute ensuite 200 ppm de tween 80 et on obtient la composition d'IgG formulée et concentrée à 250 g/ L.

### 1.12. Préparation de la composition F12

Une composition d'IgG a été obtenue selon la méthode développée par la Demanderesse dans la demande de brevet internationale WO2007/077365.
On ajoute à la composition d'IgG obtenue 250 mM de glycine et on soumet la composition d'IgG préformulée à une ultrafiltration tangentielle sur cassette à pH compris entre 4,5 et 5,5 On ajoute ensuite 200 ppm de tween 80 et on obtient la composition d'IgG formulée et concentrée à 250 g/ L.

Les résultats sont présentés dans le tableau 1 suivant.

**Tableau 1 : Compositions testées**

| | **Composition en excipients** | | | | | | | | | **pH** |
|---|---|---|---|---|---|---|---|---|---|---|
| | **IgG (g/L)** | **Glycine (mM)** | **Proline (mM)** | **Histidine (mM)** | **Arginine (mM)** | **NaCL (mM)** | **tampon acétate (mM)** | **Tween 80 (ppm)** | **Poloxamer (ppm)** | |
| **F1** | 250 | 150 | | | | 50 | | | | 4,6 -5 |
| **F2** | 250 | | 200 | | | | | | | 4,6 -5 |
| **F3** | 250 | | 200 | | | | | 200 | | 4,6 -5 |
| **F4** | 250 | 150 | | | | | 50 | 200 | | 4,6 -5 |
| **F5** | 250 | 150 | | | | | 50 | | 200 | 4,6 -5 |
| **F6** | 250 | 150 | | | | | 50 | | | 4,6 -5 |
| **F7** | 250 | | 150 | | | | 50 | 200 | | 4,6 -5 |
| **F8** | 250 | | 250 | | - | | | 200 | | 4,6 -5 |
| **F9** | 250 | - | - | 250 | - | - | - | 200 | - | 6,8 -7,8 |
| **F10** | 250 | - | - | - | 250 | | | 200 | - | 6.8 -7.8 |
| **F11** | 250 | 200 | | | | | 50 | 200 | | 4,5-5 |
| **F12** | 250 | 250 | | | | | | 200 | | 4,5-5,5 |

On utilise comme témoin, une solution IgG 10% (obtenue selon la méthode développée par la Demanderesse dans la demande de brevet internationale WO2007/077365 ou WO02/092632) et dont la composition est détaillée dans le Tableau 2 suivant.

**Tableau 2 : Témoin utilisé :**

| **Témoin** | **IgG (g/L)** | **Glycine (mM)** | **NaCL (mM)** | **Acétate de sodium (mM)** | **Tween 80 (ppm)** | **Poloxamer (ppm)** | **pH** |
|---|---|---|---|---|---|---|---|
| **IgG 10 %** | 100 | 93 | 176 | | | 50 | 4,6 - 5 |

### Exemple 2 : Etude de stabilité des compositions

### Matériels et méthodes :

### 2.1. Mesures de l'agrégation

### 2.1.1. Particules visibles

Les agrégats de protéines ou les particules exogènes de diamètre supérieur à environ 50 µm sont visibles à l'oeil nu. Dans une pièce noire, les flacons sont mirés sous un faisceau de lumière blanche par 3 opérateurs différents qui notent la présence ou l'absence de particules visibles.

### 2.1.2. Particules sub-visibles

Les particules de diamètre supérieur à 10pm et 25pm sont quantifiées par la méthode d'obscuration de lumière (Light Obscuration ; sur appareil *Model LS-200* de Particles Measuring Systems Inc.) adaptée de la Pharmacopée Européenne 6^{ème} édition, §2.9.19, méthode 1B : les échantillons sont dilués d'un facteur 2 avec de l'eau pour préparation injectable filtrée sur 0,22µm juste avant analyse pour réduire la viscosité du produit ; 5mL de produit dilué sont utilisés pour le rinçage et 5mL pour la mesure.

### 2.1.3. Mesures de diffusion dynamique de la lumière, (Dynamic Light Scattering), ci-après (DLS)

La DLS permet de mesurer les diamètres hydrodynamiques des protéines et des agrégats présents en solution. Cette mesure permet de suivre les phénomènes d'agrégation à des stades précoces de formation, puisque les tailles accessibles vont du nanomètre au micron. Pour cette étude, la mesure a été réalisée à l'aide du banc de diffusion (*ALV*/*CGS-3 Compact Goniometer System* de ALV) à un angle de 90°. Par ailleurs, 0,04M de NaCl ont été ajoutées à toutes les solutions pour maintenir une force ionique suffisante et permettre une mesure de taille cohérente.

### 2.1.4. Quantification des polymères par HP-SEC (High Pressure-Size Exclusion Chromatography)

**Il** s'agit d'une méthode de chromatographie en phase liquide qui consiste à séparer les protéines selon leur taille moléculaire et permettant de déterminer le pourcentage de monomères, dimères et polymères. Cette étude est réalisée sur une colonne *Tricorn Superdex 200 10*/*300 GL* de GE Healthcare.

### 2.2. Mesure de la fragmentation.

La fragmentation des Immunoglobulines est quantifiée par HP-SEC.

### Résultats :

Toutes les formulations présentent une bonne stabilité à 25°C.
Ces résultats montrent que la présence de tensio-actif dans la stabilité des formulations est très importante vis-à-vis de l'agrégation sous forme de microparticules (particules visibles, sub-visibles). En effet, les niveaux de particules visibles et sub-visibles détectés dans les compositions F1, F2 et F6 sont bien plus élevés que ceux mesurés dans les compositions contenant des tensio-actifs (F3, F4, F5 et F7), ce qui montre bien l'importance de la présence de tensioactifs. Après 16 mois de stabilité à 25°C, les compositions contenant des tensio-actifs (F3, F4, F5 et F7) restent exemptes de particules visibles et présentent moins de particules sub-visibles que les compositions F1, F2 et F6.
De plus, à 25°C et jusqu'à 16 mois de stabilité, le niveau des polymères détecté est en dessous des normes imposées par la pharmacopée (<3%) pour toutes les compositions.
A 25°C et jusqu'à 16 mois de stabilité, le niveau de fragmentation est comparable au produit de référence IgG 10%.

### Exemple 3 : Influence de la température sur la concentration par ultrafiltration

L'étude de l'influence de la température sur la concentration des IgG par ultrafiltration tangentielle a été réalisée sur deux sortes de membranes Ultracel V® et Ultracel C® de Millipore en cellulose régénérés à seuil de coupure de 30kD, à 15 et 20°C pour la membrane Ultracel V® et 15 et 30°C pour la membrane Ultracel C®.

### Résultats

Les résultats sont présentés dans le tableau 3 suivant.

**Tableau 3 : Concentrations sur Ultracel C® et Ultracel V® à différentes températures**

| Membranes | Ultracel V® | | Ultracel C® | |
|---|---|---|---|---|
| Températures | 15°C | 20°C | 15°C | 30°C |
| Concentrations finales obtenues (g/l) | 274,0 | 271,9 | 262,9 | 272,3 |

La faisabilité de la concentration à 250 g/l est démontrée à des températures inférieures à 30°C.

### Exemple 4 : Etude de la tolérance locale chez le mini-porc.

L'étude de tolérance locale a porté sur trois animaux traités par administration simultanée en sous-cutanée de la composition F4, d'IgNG 10%, de Subcuvia® (IgG 160g/L ou 16%, de Baxter) et de tampon de formulation glycine-acétate-tween 80, à un débit de 10 ml/h. Le volume de perfusion a été fixé à 10 ml. Une aiguille de 27 G a été utilisée. Pour éviter un biais lié au site d'injection, les sites d'injection ont été randomisés d'un animal à l'autre.

### Résultats :

Aucun signe clinique indiquant une toxicité systémique n'a été observé. Au niveau des sites d'injection, aucun hématome ou induration n'a été reporté avant, pendant ou après le traitement. Les réactions locales ont été limitées à des érythèmes et oedèmes au niveau des sites d'injection.
A l'autopsie, (examen macroscopique *post-mortem*), aucune anomalie pertinente n'a été observée.
En conclusion, dans les conditions étudiées, la tolérance locale de la composition F4 est considérée comme satisfaisante.

## Revendications

1. Procédé de préparation d'une composition pharmaceutique liquide comprenant des immunoglobulines G humaines (IgG), comprenant les étapes suivantes:
a) Fournir une préparation d'IgG;
b) Ajouter à la préparation, au moins un tensioactif et/ou au moins un acide aminé;
c) Procéder à une concentration des IgG par ultrafiltration,
d) Puis ajouter un tensioactif, qui peut être identique ou différent du tensioactif de l'étape b).

2. Procédé de préparation d'une composition pharmaceutique liquide selon la revendication 1, **caractérisé en ce que** l'on ajoute à l'étape b) au moins un acide aminé qui est un acide aminé hydrophile ou portant une chaîne latérale chargée positivement l'acide aminé étant de préférence de la glycine, de préférence à une concentration de 200 à 300mM.

3. Procédé de préparation d'une composition pharmaceutique liquide selon l'une des revendications 1 à 2, **caractérisé en ce qu'**on ajoute en outre à l'étape b) un ou plusieurs composés choisis parmi les sucres, les dérivés de sucres, et les sels, ledit sel étant de préférence choisi parmi un sel de métal alcalin, alcalino-terreux, ou d'un métal de transition, choisi parmi un sel minéral et un sel organique, de préférence de 0 à 100mM d'acétate de sodium, et ledit sucre étant de préférence choisi parmi le saccharose, les di- et tri-saccharides et les polysaccharides.

4. Procédé de préparation d'une composition pharmaceutique liquide selon les revendications 1 à 3, **caractérisé en ce que** le tensioactif ajouté à l'étape d) est de préférence un détergent non ionique.

5. Procédé de préparation d'une composition pharmaceutique liquide selon les revendications 1 à 4, **caractérisé en ce que** le tensioactif est choisi parmi du polysorbate et un poloxamer, de préférence un polysorbate, de préférence du polysorbate 20 ou du polysorbate 80.

6. Procédé selon l'une des revendications 1 à 5, **caractérisé en ce que** le tensioactif est ajouté aux étapes b) et/ou d), à une concentration totale de 50 à 300ppm.

7. Procédé de préparation d'une composition pharmaceutique liquide selon les revendications 1 à 6, **caractérisé en ce que** le tensioactif est ajouté à l'étape b) à une concentration inférieure à 75ppm, de préférence inférieure à 50ppm, de préférence inférieure à 40ppm.

8. Procédé de préparation d'une composition pharmaceutique liquide selon l'une des revendications 1 à 7, **caractérisé en ce que** l'ultrafiltration de l'étape c) est réalisée à une température inférieure à 30°C, de préférence inférieure à 25°C, de préférence encore à une température entre 15°C et 25°C, de préférence environ 20°C.

9. Procédé de préparation d'une composition pharmaceutique liquide selon l'une des revendications 1 à 8, **caractérisé en ce que** la concentration en IgG est d'au moins 230g/L, de préférence comprise entre 230g/L et 350g/L, dans ladite composition.

10. Composition pharmaceutique liquide susceptible d'être obtenue par le procédé selon l'une des revendications 1 à 9, dans laquelle la composition pharmaceutique obtenue est à un pH compris entre 4,2 et 5,5 , ou à un pH compris entre 6,8 et 7,8.

11. Composition pharmaceutique liquide comprenant
- Immunoglobulines G humaines concentrées entre 230 et 350g/L, de préférence à environ 250g/L, de préférence à environ 250g/L ;
- glycine ;
- éventuellement du tampon acétate de sodium;
- du polysorbate, de préférence du polysorbate 80, ou un poloxamer,
ladite composition comprenant de préférence
- Immunoglobulines G humaines concentrées entre 230 et 350g/L, de préférence à environ 250g/L ;
- 200 à 300 mM de glycine ;
- 0 à 100 mM de tampon acétate de sodium;
- et 50 à 300 ppm de polysorbate.

12. Composition pharmaceutique liquide selon la revendication 11 comprenant
- Immunoglobulines G humaines concentrées à environ 250g/L ;
- 200 mM de glycine ;
- 50 mM de tampon acétate de sodium;
- et 200 ppm de polysorbate.
ou comprenant
- Immunoglobulines G humaines concentrées à environ 250g/L ;
- 250 mM de glycine ;
- et 200 ppm de polysorbate.
ou comprenant, le pH de la composition étant alors compris entre 4,2 et 5,5:
- IgG à environ 250g/l ;
- environ 150 mM glycine;
- environ 50 mM de tampon acétate de sodium
- et environ 200 ppm de polysorbate 80

13. Composition pharmaceutique liquide comprenant des immunoglobulines G humaines (IgG), **caractérisée en ce que** la concentration en IgG est d'au moins 230 g/L, de préférence entre 230 et 350g/Lde préférence à pH compris entre 6,8 et 7,8, de préférence encore à pH 7,3.

14. Composition pharmaceutique liquide selon l'une quelconque des revendications 10 à 13, sous une forme adaptée pour une administration sous cutanée ou intramusculaire, de préférence une administration sous-cutanée.

15. Composition pharmaceutique liquide selon l'une quelconque des revendications 10 à 14, comprenant des immunoglobulines G humaines (IgG) pour une utilisation comme médicament afin de traiter les déficits immunitaires primitifs avec défaut de production d'anticorps, la maladie de Kawasaki, le purpura thrombopénique immunologique de l'enfant et de l'adulte, les déficits immunitaires secondaires avec défaut de production d'anticorps, en particulier la leucémie lymphoïde chronique ou myélome associés à des infections à répétition, l'infection de l'enfant par le VIH associé à des infections bactériennes, les neuropathies motrices multifocales, le syndrome de Guillain-Barré, les infections aiguës sévères ou chroniques à Parvovirus B19, l'immunodéficience acquise ou constitutionnelle, la dermatomyosite cortico-résistante, la myasthénie aiguë, la polyradiculonévrite chronique idiopathique, le purpura thrombopénique immunologique, par exemple associé à l'infection par le VIH, le syndrome de l'homme raide (Stiffman syndrome), la neutropénie auto-immune, l'erythroblastopénie auto-immune résistante, le syndrome d'anti-coagulation acquise par auto-anticorps, la polyarthrite rhumatoïde, les uvéites.
